# EUROPEAN PATENT SPECIFICATION

(11) **EP 2 023 980 B1**
(45) Date of publication and mention of the grant of the patent: **29.05.2019**
(21) Application number: 07733008.2
(22) Date of filing: 30.05.2007
(51) Int. Cl.: A61M 5/50, A61M 5/20, A61M 5/32

(54) **INJECTION DEVICE WITH A DAMPING MEANS OF THE TRIGGER**
INJEKTIONSGERÄT MIT TRIGGER-DÄMPFUNGSMITTEL
DISPOSITIF D'INJECTION AVEC DES MOYENS D'AMORTISSEMENT DU DÉCLENCHEUR

(30) Priority: 01.06.2006 GB 0610860
(43) Date of publication of application: 18.02.2009
(73) Proprietor: Cilag GmbH International, 6300 Zug (CH)
(72) Inventor: HARRISON, Nigel, Linton, Cambridge CB1 6YN (GB); JENNINGS, Douglas, Royston, Hertfordshire SG8 7XU (GB)
(74) Representative: Tunstall, Christopher Stephen
(86) International application number: PCT/GB2007/001992
(87) International publication number: WO 2007/138313

(56) References cited:
- WO-A-03/047663
- WO-A-2005/115509
- WO-A-2005/115512
- US-B1- 6 270 479

## Description

### FIELD OF THE INVENTION

The present invention relates to an injection device of the type that receives a syringe, extends it, discharges its contents and then retracts it automatically.

### BACKGROUND OF THE INVENTION

Previously known injection devices are shown in WO 95/35126 and EP-A-0 516 473 and tend to employ a trigger that, when a releasable locking mechanism allows its activation, may be operated to cause a drive spring to act upon a syringe.

Generally, the trigger is rotatable about a pivot axis so that when it is depressed at a first end, a second end (that normally engages the drive spring) is also rotated, thereby releasing the drive spring, extending the syringe and discharging its contents. When the releasable locking mechanism is engaged, the rotation of the trigger is unhindered and it may be activated. When the releasable locking mechanism is not engaged, it acts to prevent rotation of the trigger and release of the drive spring. This way, accidental activation of the trigger can be prevented.

A problem with an injection device of this type is that the trigger may be unintentionally activated by a sudden impact force, for example that associated with the injection device being dropped and striking the ground. Contrary to the design intent, interlocks in the device can be defeated by such a force and activation of the trigger becomes allowed, and may indeed occur as a direct result of the impulse. A further problem is that the trigger can be loose and free to move after its actuation and thus can return to its starting position, therefore making it unclear as to whether the device has already been used or not.

WO2005/115509A1 discloses an injection device with a housing adapted to receive a syringe.

US6270479B1 discloses an autoinjector for replaceable containers of syringe type.

WO03/047663A discloses an automatic injector with a needle that is injected automatically into the injection site.

WO2005/115512A1 discloses an injection device.

### SUMMARY OF THE INVENTION

The injection device of the present invention is designed to overcome this and other problems.

In view of the foregoing and in accordance with a first aspect of the invention, there is provided an injection device according to claim 1 comprising:
a housing adapted to receive a syringe having a discharge nozzle, so that the syringe is movable between a retracted position in which the discharge nozzle is contained within the housing and an extended position in which the discharge nozzle extends from the housing through an exit aperture;
a drive that is acted upon and in turn acts upon the syringe;
a trigger movable from a rest position, in which it causes the drive to be retained, to an active position, in which it no longer causes the drive to be retained; and
a damping means on the trigger.

The damping means absorbs energy from an impact and reduces its transmission to the trigger. In this way, the damping means prevents accidental activation of the device that could otherwise occur if, for example, it was dropped onto a hard surface. Provision of the damping means has the additional advantages of holding the trigger in its active position after its actuation, so that it is clear when the device has been used; and of reducing noise when the trigger is operated.

The damping means acts between the trigger and the housing to resist motion of the trigger.

The trigger is pivotally mounted along a pivot axis and the damping means may then act between the trigger and the housing to resist rotational motion of the trigger.

The drive is acted upon by a biasing means, this is preferably a spring. Accordingly, an end of the trigger causes the drive to be retained when the trigger is in its rest position.

Preferably, the damping means is positioned between the pivot axis and the end of the trigger that causes the drive to be retained.

Advantageously, the damping means is adapted to retain the trigger in its active position after actuation. This thereby serves as an indication that the injection device has been used.

The damping means is be a viscous fluid. Preferably, the viscous fluid is a non-Newtonian fluid. The viscous fluid is preferably non-toxic and not damaging to plastics.

The trigger may include at least one opening in contact with the viscous fluid. The at least one opening is preferably positioned between an upper surface and a lower surface of the trigger.

The viscous fluid is positioned in a region between the upper surface of the trigger and an inner surface of the housing.

Advantageously, the at least one opening is dimensioned to permit the viscous fluid to escape from the region between the upper surface of the trigger and an inner surface of the housing.

The consistency of the viscous fluid of the present invention may be characterised by its unworked penetration. Penetration is defined as the depth to which a standard penetrator, such as a cone or a needle, sinks under gravity into a semi-solid substance under defined conditions of sample size, penetrator weight and geometry and time (typically five seconds). The penetration values quoted herein are taken as having been measured under the conditions prescribed by ISO 2137.

The consistency of the viscous fluid is advantageously such that the viscous fluid will be retained in place on the trigger prior to its activation but may also leave the region of its confinement, via the openings, when the trigger is operated by a user.

The viscous fluid may have an unworked penetration of between 16 mm and 31 mm. Preferably, the viscous fluid has an unworked penetration of between 17.5 mm and 21 mm. More preferably, the viscous fluid has an unworked penetration of between 19 mm and 20 mm. A viscous fluid according to the present invention may be Molykote® 111.

Advantageously, the damping means prevents accidental actuation of the trigger.

### BRIEF DESCRIPTION OF THE DRAWINGS

The invention will now be described by way of example with reference to the accompanying drawings, in which:
Figure 1 shows a perspective view of an injection device according to the present invention;
Figure 2 shows a side view of the injection device of figure 1 with the housing of the injection device removed;
Figure 3 shows a perspective view of the trigger and an upper section of the housing of the injection device of figure 1;
Figure 4 shows a bottom view of the trigger and an upper section of the housing of the injection device of figure 1; and
Figure 5 shows a side view of the injection device of figure 1 displaying the positioning of the damping means between the trigger and the upper section of the housing.

### DETAILED DESCRIPTION OF THE DRAWINGS

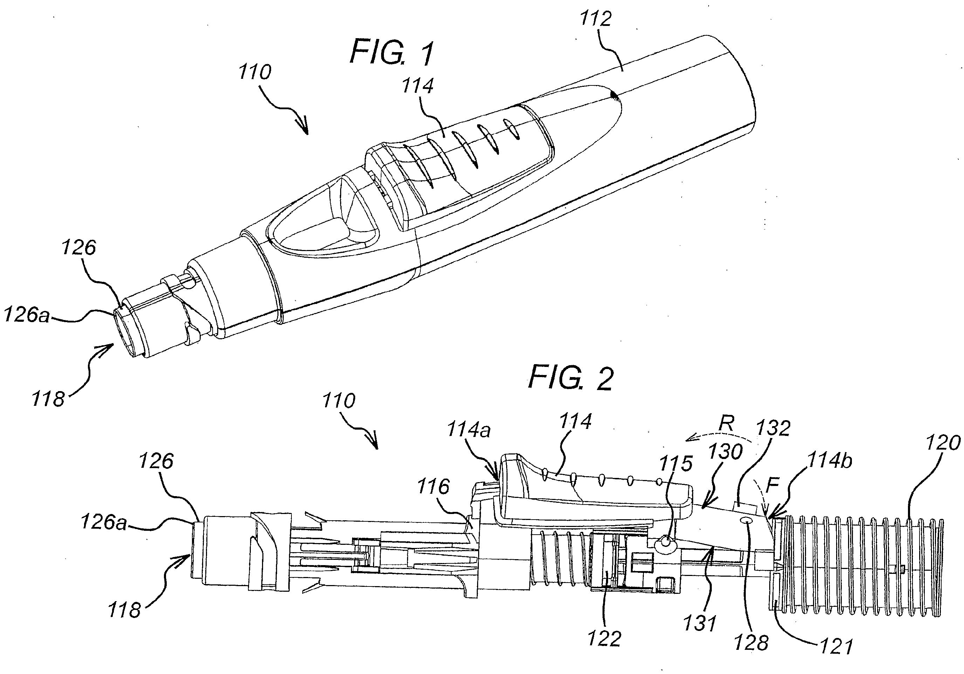

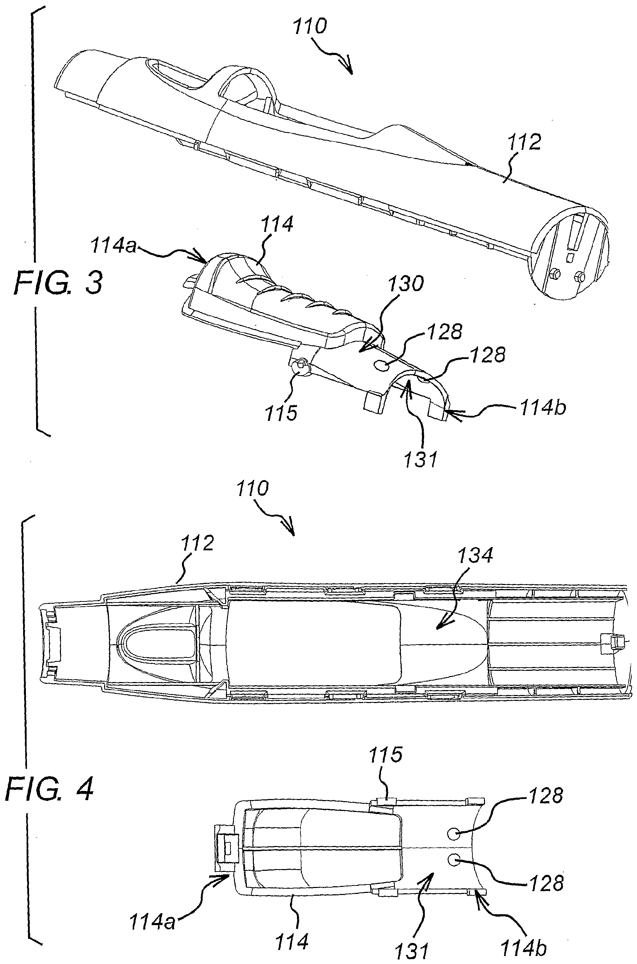

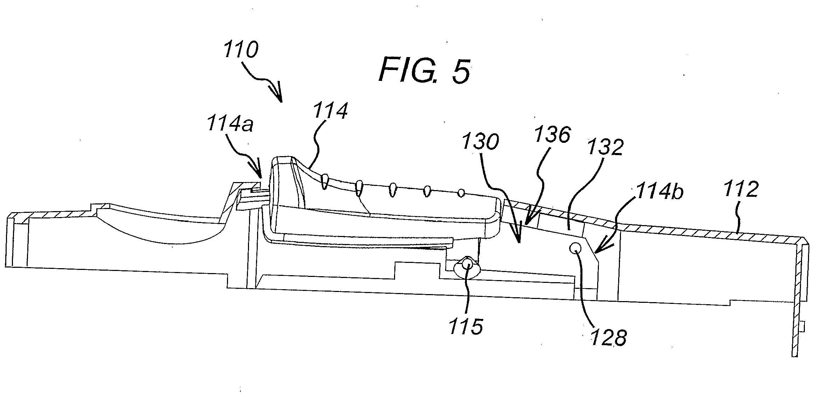

Figures 1 to 5 show an injection device 110 according to a first embodiment of the present invention. The injection device 110 has an injection device housing 112.

A syringe 122 is contained within the housing 112. The injection device 110 comprises a trigger 114 and a releasable locking mechanism 116. The trigger 114 has a first end 114a and a second end 114b. The trigger 114 is rotatable about a pivot 115 from a rest position to an active position. The second end 114b of the trigger 114 connects with a drive coupling 121, which is acted upon by a drive spring 120. The drive coupling 121 is in communication with the syringe 122.

Rotation of the trigger 114 about the pivot 115 in a direction R (i.e. downwards into the housing 112 at its first end 114a) causes the second end 114b of the trigger 114 to disengage from the drive coupling 121, thereby letting the drive spring 120 drive the syringe 122 (via the drive coupling 121) out of an aperture 118 in the housing 112.

The releasable locking mechanism 116 is in communication with the sliding sleeve 126 which protrudes, when in a first position, from the aperture 118 in the housing 112. The releasable locking mechanism 116 is deactivated by movement of the sliding sleeve 126 into the housing 112 into a second position.

A first end 126a of the sliding sleeve 126 can be placed against a body into which a drug is being delivered, thereby deactivating the releasable locking mechanism 116 and allowing the trigger 114 to rotate in a direction R from its rest position to its active position.

The trigger 114 includes openings 128 that run from an upper surface 130 to a lower surface 131. The openings 128 are positioned between the second end 114b of the trigger 114 and the pivot 115. A damping means 132 is positioned between the upper surface 130 of the trigger 114 and an inner surface 134 of the housing 112 and over the openings 128. The damping means 132 is a viscous fluid that has a viscosity sufficient to retain it in place between the housing 112 and the trigger 114 during normal usage of the device 110.

Rotation of the trigger 114 in the direction R about the pivot 115 will result in the first end 114a moving down into the housing 112 and the second end 114b moving up toward the inner surface 134 of the housing 112. The openings 128 and the damping means 132 act between the inner surface 134 of the housing 112 and the upper surface 130 of the trigger 114 to provide a resistive moment of force F to rotation in the direction R.

Upon normal operation of the trigger 114 by a user, the viscous fluid becomes compressed between the upper surface 130 of the trigger 114 and the inner surface 134 of the housing 112, thereby offering resistance to the motion of the trigger 114. The viscous fluid may then leave a confined area 136 that is bordered by these surfaces via openings 128. Rotation of the trigger 114 in the direction R then becomes possible as the viscous fluid leaves the confined area 136 and the device 110 operates as described above. Following the operation of the trigger 114, most of the viscous fluid becomes positioned on the lower surface 131 of the trigger 114. A residual amount of viscous fluid remains on the upper surface 130 of the trigger 114 in contact with the inner surface 134 of the housing 112. Due to its viscosity, the viscous fluid has certain adhesive properties that cause the trigger 114 to be retained in its active position, thereby indicating that the device 110 has been used.

Unintentional operation of the injection device 110 is prevented by the damping means 132. If the interlocks provided by the sliding sleeve 126 and the releasable locking mechanism 116 are defeated by an impact force, for example as a result of the device being dropped onto a hard surface, the damping means 132 provides resistance to rotational motion of the trigger 114 in the direction R. The damping means 132 also acts to reduce transmission of such an impact force to the trigger 114, preventing the device 110 from self-actuating. The arrangement of the openings 128 and the damping means 132 thereby allows the device 110 to operate in a more reliable manner. The provision of the damping means 132 has the additional benefit of limiting damage to the device 110 if it were dropped onto a hard surface. It also reduces any noise produced by the operation of the trigger 114 that might be distressing to a user of the device 110.

It will of course be understood that the present invention has been described above purely by way of example and modifications of detail can be made within the scope of the invention.

## Claims

1. An injection device (110) comprising:
a housing (112) adapted to receive a syringe having a discharge nozzle, so that the syringe is movable between a retracted position in which the discharge nozzle is contained within the housing and an extended position in which the discharge nozzle extends from the housing through an exit aperture;
a drive coupling that is acted upon by a biasing means and in turn acts upon the syringe;
a trigger (114) movable from a rest position, in which it causes the drive to be retained, to an active position, in which it no longer causes the drive to be retained, wherein
the trigger (114) is pivotally mounted along a pivot axis and rotatable about the pivot axis from the rest position to the active position, wherein an end of the trigger (114b) causes the drive coupling to be retained when the trigger (114) is in its rest position and rotation of the trigger (114) causes the end of the trigger to disengage from the drive coupling; **characterised by**
a damping means (132) on the trigger (114), wherein the damping means (132) is a viscous fluid positioned in a region between an upper surface (130) of the trigger (114) and an inner surface (134) of the housing (112) wherein the damping means (132) acts between the trigger (114) and the housing (112) to resist motion of the trigger (114).

2. The injection device of claim 1, wherein the damping means acts between the trigger (114) and the housing (112) to resist rotational motion of the trigger (114).

3. The injection device of claim 3, wherein the biasing means is a spring (120).

4. The injection device of any preceding claim, wherein the damping means (132) is positioned between the pivot axis and the end of the trigger (114b) that causes the drive to be retained.

5. The injection device of any preceding claim, wherein the viscous fluid is adapted to retain the trigger (114) in its active position after actuation.

6. The injection device of claim 1, wherein the viscous fluid is a non-Newtonian fluid.

7. The injection device of any preceding claim, wherein the trigger (114) includes at least one opening (128) in contact with the viscous fluid.

8. The injection device of claim 7, wherein the at least one opening (128) is positioned between an upper surface (130) and a lower surface (131) of the trigger.

9. The injection device of claim 8, wherein the opening (128) is dimensioned to permit the viscous fluid to escape from the region between the upper surface (130) of the trigger and an inner surface (134) of the housing (112).

10. The injection device of any preceding claim, wherein the viscous fluid has an unworked penetration of between 16 mm and 31 mm.

11. The injection device of claim 10, wherein the viscous fluid has an unworked penetration of between 17.5 mm and 21 mm.

12. The injection device of claim 11, wherein the viscous fluid has an unworked penetration of between 19 mm and 20 mm.

## Patentansprüche

1. Injektionsgerät (110), umfassend:
ein Gehäuse (112), das dazu angepasst ist, eine Spritze mit einer Auslassdüse derart aufzunehmen, dass die Spritze zwischen einer eingefahrenen Position, in der die Auslassdüse in dem Gehäuse enthalten ist, und einer ausgefahrenen Position, in der sich die Auslassdüse von dem Gehäuse durch eine Ausgangsöffnung erstreckt, bewegt werden kann;
eine Antriebskopplung, auf die ein Vorspannmittel wirkt und die wiederum auf die Spritze wirkt;
einen Auslöser (114), der aus einer Ruheposition, in der er bewirkt, dass der Antrieb gehalten wird, in eine aktive Position, in der er nicht länger bewirkt, dass der Antrieb gehalten wird, bewegt werden kann, wobei
der Auslöser (114) schwenkbar entlang einer Schwenkachse montiert ist und aus der Ruheposition in die aktive Position um die Schwenkachse gedreht werden kann, wobei ein Ende des Auslösers (114b) bewirkt, dass die Antriebskopplung gehalten wird, wenn der Auslöser (114) in seiner Ruheposition ist, und die Drehung des Auslösers (114) bewirkt, dass das Ende des Auslösers aus der Antriebskopplung ausrückt; **gekennzeichnet durch**
ein Dämpfungsmittel (132) an dem Auslöser (114), wobei das Dämpfungsmittel (132) eine viskose Flüssigkeit ist, die in einem Bereich zwischen einer Oberfläche (130) des Auslösers (114) und einer Innenfläche (134) des Gehäuses (112) positioniert ist, wobei das Dämpfungsmittel (132) zwischen dem Auslöser (114) und dem Gehäuse (112) wirkt, um der Bewegung des Auslösers (114) einen Widerstand entgegenzusetzen.

2. Injektionsgerät nach Anspruch 1, wobei das Dämpfungsmittel zwischen dem Auslöser (114) und dem Gehäuse (112) wirkt, um der Drehbewegung des Auslösers (114) einen Widerstand entgegenzusetzen.

3. Injektionsgerät nach Anspruch 3, wobei das Vorspannmittel eine Feder (120) ist.

4. Injektionsgerät nach einem vorhergehenden Anspruch, wobei das Dämpfungsmittel (132) zwischen der Schwenkachse und dem Ende des Auslösers (114b) positioniert ist, der bewirkt, dass der Antrieb gehalten wird.

5. Injektionsgerät nach einem vorhergehenden Anspruch, wobei die viskose Flüssigkeit dazu angepasst ist, den Auslöser (114) nach Betätigung in seiner aktiven Position zu halten.

6. Injektionsgerät nach Anspruch 1, wobei die viskose Flüssigkeit eine nichtnewtonsche Flüssigkeit ist.

7. Injektionsgerät nach einem vorhergehenden Anspruch, wobei der Auslöser (114) mindestens eine Öffnung (128) in Kontakt mit der viskosen Flüssigkeit umfasst.

8. Injektionsgerät nach Anspruch 7, wobei die mindestens eine Öffnung (128) zwischen einer Oberseite (130) und einer Unterseite (131) des Auslösers positioniert ist.

9. Injektionsgerät nach Anspruch 8, wobei die Öffnung (128) derart bemessen ist, dass sie erlaubt, dass die viskose Flüssigkeit aus dem Bereich zwischen der Oberseite (130) des Auslösers und einer Innenseite (134) des Gehäuses (112) entweichen kann.

10. Injektionsgerät nach einem vorhergehenden Anspruch, wobei die viskose Flüssigkeit eine Ruhepenetration zwischen 16 mm und 31 mm aufweist.

11. Injektionsgerät nach Anspruch 10, wobei die viskose Flüssigkeit eine Ruhepenetration zwischen 17,5 mm und 21 mm aufweist.

12. Injektionsgerät nach Anspruch 11, wobei die viskose Flüssigkeit eine Ruhepenetration zwischen 19 mm und 20 mm aufweist.

## Revendications

1. Dispositif d'injection (110) comprenant :
un boîtier (112) apte à recevoir une seringue comportant un gicleur d'écoulement, de façon à ce que la seringue puisse se déplacer entre une position rentrée, dans laquelle le gicleur d'écoulement est contenu à l'intérieur du boîtier, et une position sortie, dans laquelle le gicleur d'écoulement sort du boîtier par une ouverture de sortie ;
un accouplement de transmission sur lequel agit un moyen de sollicitation et qui à son tour agit sur la seringue ;
un déclencheur (114) pouvant se déplacer d'une position de repos, dans laquelle il retient la transmission, à une position active, dans laquelle il ne retient plus la transmission,
dans lequel le déclencheur (114) est monté pivotant le long d'un axe de pivotement et peut tourner autour de l'axe de pivotement de la position de repos à la position active ;
dans lequel une extrémité (114b) du déclencheur retient l'accouplement de transmission quand le déclencheur (114) est dans sa position de repos et la rotation du déclencheur (114) fait se désaccoupler l'extrémité du déclencheur de l'accouplement de transmission,
**caractérisé par** un moyen d'amortissement (132) sur le déclencheur (114),
dans lequel le moyen d'amortissement (132) est un fluide visqueux placé dans une zone entre la surface supérieure (130) du déclencheur (114) et la surface intérieure (134) du boîtier (112) ;
dans lequel le moyen d'amortissement (132) agit entre le déclencheur (114) et le boîtier (112) pour résister au mouvement du déclencheur (114).

2. Dispositif d'injection selon la revendication 1, dans lequel le moyen d'amortissement agit entre le déclencheur (114) et le boîtier (112) pour résister au mouvement de rotation du déclencheur (114).

3. Dispositif d'injection selon la revendication 3, dans lequel le moyen de sollicitation est un ressort (120).

4. Dispositif d'injection selon l'une quelconque des revendications précédentes, dans lequel le moyen d'amortissement (132) est placé entre l'axe de pivot et l'extrémité (114b) du déclencheur qui retient la transmission.

5. Dispositif d'injection selon l'une quelconque des revendications précédentes, dans lequel le fluide visqueux est apte à retenir le déclencheur (114) dans sa position active après actionnement.

6. Dispositif d'injection selon la revendication 1, dans lequel le fluide visqueux est un fluide non newtonien.

7. Dispositif d'injection selon l'une quelconque des revendications précédentes, dans lequel le déclencheur (114) comprend au moins une ouverture (128) en contact avec le fluide visqueux.

8. Dispositif d'injection selon la revendication 7, dans lequel ladite ouverture (128) est placée entre la surface supérieure (130) et la surface inférieure (131) du déclencheur.

9. Dispositif d'injection selon la revendication 8, dans lequel l'ouverture (128) est dimensionnée pour permettre au fluide visqueux de s'échapper de la zone entre la surface supérieure (130) du déclencheur et la surface intérieure (134) du boîtier (112).

10. Dispositif d'injection selon l'une quelconque des revendications précédentes, dans lequel le fluide visqueux présente une pénétration sans malaxage comprise entre 16 mm et 31 mm.

11. Dispositif d'injection selon la revendication 10, dans lequel le fluide visqueux présente une pénétration sans malaxage comprise entre 17,5 mm et 21 mm.

12. Dispositif d'injection selon la revendication 11, dans lequel le fluide visqueux présente une pénétration sans malaxage comprise entre 19 mm et 20 mm.
